# FASCICULE DE BREVET EUROPEEN

(11) **EP 0 912 492 B1**
(45) Date de publication et mention de la délivrance du brevet: **25.09.2002**
(21) Numéro de dépôt: 97934576.6
(22) Date de dépôt: 16.07.1997
(51) Int. Cl.: C07C 209/74, C07C 231/12

(54) **PROCEDE DE PREPARATION DE DERIVES HALOGENES DU 2-AMINO OU DU 2-ACETAMIDO TRIFLUOROMETHYLBENZENE**
VERFAHREN ZUR HERSTELLUNG VON HALOGENIERTEN DERIVATEN DES 2-AMINO ODER 2-ACETAMIDO TRIFLUORMETHYLBENZOLS
METHOD FOR PREPARING HALOGENATED 2-AMINO OR 2-ACETAMIDO TRIFLUOROMETHYLBENZENE DERIVATIVES

(30) Priorité: 16.07.1996 FR 9608885
(43) Date de publication de la demande: 06.05.1999
(73) Titulaire: RHODIA CHIMIE, 92408 Courbevoie Cédex (FR)
(72) Inventeur: DURY, Michel, F-69006 Lyon (FR)
(74) Mandataire: Bernasconi, Jean Raymond
(86) Numéro de dépôt international: FR9701323
(87) Numéro de publication internationale: WO98002409

(56) Documents cités:
- FR-A- 800 343
- US-A- 2 093 115
- US-A- 4 008 278
- PATENT ABSTRACTS OF JAPAN vol. 012, no. 306 (C-522), 19 août 1988 & JP 63 077844 A (NIPPON KAYAKU CO LTD), 8 avril 1988,
- PATENT ABSTRACTS OF JAPAN vol. 006, no. 085 (C-103), 22 mai 1982 & JP 57 018638 A (SAGAMI CHEM RES CENTER), 30 janvier 1982, cité dans la demande

## Description

La présente invention concerne un procédé de préparation de dérivés du 2-amino ou 2-acétamido trifluorométhylbenzène chlorés en position 5 sur le cycle benzénique.

Le 5-chloro 2-aminotrifluorométhylbenzène est un intermédiaire de synthèse utilisé notamment dans la fabrication de colorants, ou d'un fongicide, le triflumizole.

De nombreuses voies de synthèse ont été proposées pour atteindre ce composé le plus souvent en au moins deux étapes à partir d'un précurseur simple.

Ainsi, le brevet FR-A-800 343 décrit la préparation du 5-chloro 2-aminotrifluorométhylbenzène à partir du 3-chlorotrifluorométhylbenzène par nitration dans un mélange acide nitrique-acide sulfurique, suivie d'une réduction.

Plus récemment, des procédés en une seule étape ont été proposés. Le brevet JP-A-63 077 844 décrit la préparation de la 4-bromo-2-fluoro-5-trifluorométhylamine pas bromation dans le chlorure méthylène du dérivé aniline correspondant. Le brevet JP-A-57 018 638 du 07.07.8.0 décrit la trifluorométhylation de la p-chloroaniline.

La chloration du 2-amino trifluorométhyl benzène a également été envisagée mais il est difficile d'obtenir avec une sélectivité suffisante le produit de chloration désiré.

Dans le brevet US-A-4 008 278 est présentée une solution pour éviter la formation d'autres produits de monochloration que le 5-chloro 2-aminotrifluorométhylbenzène. Elle consiste à faire réagir le 2-aminotrifluorométhylbenzène, ou un dérivé N-acétylé ou N-formylé, avec de l'acide chlorhydrique en présence d'un oxydant.

Le réactif de départ est converti à 64,5 % en 5-chloro 2-aminotrifluorométhylbenzène, à 34,2 % en 3,5-dichloro 2-aminotrifluorométhylbenzène et on ne relève que des traces de 3-chloro 2-amino trifluorobenzène.

La sélectivité de la réaction dans ces conditions reste cependant insuffisante car on obtient les produits de mono- et dichloration dans un rapport de l'ordre de 2:1. Cela impose une étape finale de séparation des isomères dans laquelle il faut traiter un volume important de produit réactionnel pour isoler le composé désiré. La productivité de tels procédés est donc faible.

Le but de l'invention est de proposer des conditions opératoires imposant une sélectivité élevée à la réaction de chloration du 2-amino ou du 2-acétamido trifluorométhylbenzène pour une productivité améliorée.

D'autres aspects de l'invention en relation avec cet objectif apparaîtront par la suite.

Il s'est révélé qu'une sélectivité améliorée pouvait être atteinte en effectuant la chloration sous l'action du Cl₂ dans un solvant déterminé.

Ainsi, l'invention a pour objet un procédé de préparation de 2-amino ou 2-acétamido trifluorométhylbenzène halogéné en position 5, comprenant une étape de chloration d'un composé de formule (1) 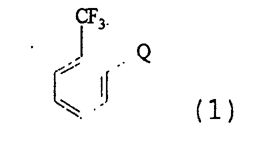 dans laquelle Q représente un groupe NH₂ éventuellement sous forme d'un sel d'addition avec un acide tel que l'acide chlorhydrique, ou un groupe NHCOCH₃,
au moyen de Cl₂,
dans un solvant choisi parmi un hydrocarbure halogéné anhydre, un mélange d'eau et d'hydrocarbure halogéné et le-composé de formule (1) lui-même,
pour donner majoritairement un composé de formule (2) ou (3) 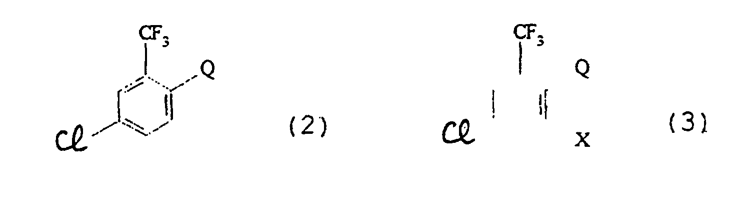 formules dans lesquelles Q est tel que défini ci-dessus.

Le produit de départ de formule (1) peut être le 2-amino trifluorométhylbenzène (Q = NH₂), dans lequel le groupe amino est éventuellement sous forme d'un sel d'addition avec un acide connu en soi, tel que notamment l'acide chlorhydrique, ou encore le 2-acétamido trifluorométhylbenzène (Q = NHCOCH₃).

Ces composés présentent une réactivité sensiblement identique vis-à-vis de l'halogénation dans les conditions de l'invention.

La réaction de chloration selon la présente invention ne nécessite pas la présence d'une base organique.

Il est apparu que le choix du solvant permet d'orienter la régiosélectivité de l'introduction du ou des atomes d'halogène.

Ainsi, lorsque la réaction est conduite dans un hydrocarbure halogéné anhydre, on obtient majoritairement le produit de formule (2). L'isomère de monochloration en position 3 n'est présent à la fin de la réaction qu'à l'état de traces.

En fait, il a été mis en évidence que ce composé peut se former au cours de la réaction mais est totalement converti en composé dichloré de formule (3) avec l'avancement de la réaction. Le composé de formule (3) ne se forme qu'en quantité limitée, les rendements en composés (2) et (3) sont dans un rapport molaire (2)/(3) d'au moins 3:1.

Pour garantir une sélectivité maximale et éviter la présence d'isomère monochloré en position 3, il est préférable d'opérer à conversion complète ou presque complète. Avantageusement, la réaction d'halogénation est conduite jusqu'à ce que le taux de transformation du composé de formule (1) soit d'au moins 80 %, de préférence d'au moins 85 %, et même d'au moins 90 % (le taux de transformation TT étant le rapport molaire de la quantité de composé (1) disparue au cours de la réaction sur la quantité de composé (1) initiale).

On parvient, de manière générale, à un rendement de transformation du composé de formule (1) en composé de formule (2) de l'ordre de 70 % ou plus, avantageusement d'au moins 75 % et même d'au moins 80 % (le rendement de transformation RT de (1) en (2) étant le rapport molaire de la quantité de composé (2) formée sur la quantité de composé (1) disparue).

Concernant les hydrocarbures halogénés appropriés, il peut s'agir aussi bien d'hydrocarbures aliphatiques ou aromatiques. On pourra citer notamment le dichlorométhane, le monochlorobenzène, le dichlorobenzène ou encore le trifluorométhylbenzène.

De manière avantageuse, le Cl₂ est introduit de façon progressive dans le solvant réactionnel pendant la réaction, éventuellement par portions, de préférence en continu.

Avantageusement, la quantité de Cl₂ introduite dans le milieu réactionnel est telle que le rapport molaire de la quantité de Cl₂ introduite à la quantité de composé de formule (1) est de 1 à 2 environ.

Le composé de formule (1) peut être introduit en une seule fois dans le mélange réactionnel au début de la réaction, ou bien être introduit de façon progressive tout comme le réactif d'halogénation au cours de la réaction.

Suivant une variante de l'invention, il est possible de conduire la réaction d'halogénation sans solvant ou, plus exactement, dans un solvant constitué du composé de formule (1) lui-même.

Dans ces conditions, on forme également de façon majoritaire le composé de formule (2) avec un rendement de transformation de (1) en (2) de l'ordre de 70 % ou plus, notamment d'au moins 75 %, avantageusement d'au moins 80 %.

Pour qu'une partie du composé de formule (1) puisse jouer le rôle de solvant, ce composé doit être en relatif excès par rapport au réactif d'halogénation introduit. De manière avantageuse, le rapport molaire de la quantité de Cl₂ introduite à la quantité initiale de composé de formule (1) est de l'ordre de 0,1 à 0,5.

Le taux de transformation du composé de formule (1) sera d'autant plus limité que ce dernier sera en excès. A la différence de la variante précédente, du fait de la transformation incomplète, on obtient un mélange des isomères monochlorés majoritairement en position 5 et minoritairement en position 3.

Cependant, en raison de la quantité plus importante de composé (1) susceptible de réagir dans un volume de réacteur donné et du rendement de transformation (sélectivité) très élevé en faveur de l'isomère 5-chloré, la productivité en cet isomère est excellente, de l'ordre de 300 kg/m³ de mélange réactionnel. Dans ces conditions, la séparation des isomères par distillation ne grève pas excessivement le coût de production et donc la productivité globale est satisfaisante.

Suivant une troisième variante, on peut totalement renverser la sélectivité en faveur du composé dichloré de formule (3) en choisissant un solvant constitué d'un mélange d'eau et .d'un hydrocarbure halogéné, tel que ceux décrits précédemment, et en introduisant le composé de formule (1), de même que le réactif Cl₂, de façon progressive, avantageusement continue, dans le solvant réactionnel.

De préférence, le solvant contient au moins 20 %, par exemple de 20 à 80 % en poids d'hydrocarbure halogéné, avantageusement au moins 30 %, notamment environ 50% en poids.

Pour réaliser la chloration, la quantité de Cl₂ introduite est avantageusement de l'ordre de 1,5 à 2,5 mol par mol de composé de formule (1).

Le rendement de transformation en le composé de formule (3) est d'au moins 70 %, avantageusement de 75 % ou plus.

La réaction de chloration dans ces trois variantes a lieu avantageusement à une température de l'ordre de 5 à 80°C. De préférence, lorsque le composé de départ de formule (1) est le 2-amino trifluorométhylbenzène ou un sel d'addition en dérivant, la température du milieu réactionnel est de 5 à 50°C, avantageusement de 5 à 20-25°C, et lorsque le composé de départ de formule (1) est le 2-acétamido trifluorométhylbenzène, la température du milieu réactionnel est de 70 à 80°C.

Comme il a été dit précédemment, la présente invention fournit les moyens de réaliser de façon complète ou presque complète la réaction de chloration d'un dérivé du 2-amino ou du 2-acétamido trifluorométhylbenzène halogéné en position 3 pour donner les dérivés dichlorés de formule (3).

A cet égard, l'invention a également pour objet un procédé de préparation d'un dérivé dichloré du 2-amino ou du 2-acétamido trifluorométhylbenzène, comprenant une étape de chloration d'un composé de formule (2') 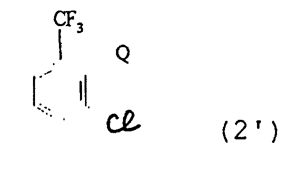 dans laquelle Q représente un groupe NH₂ éventuellement sous forme d'un sel d'addition avec un acide tel que l'acide chlorhydrique, ou un groupe NHCOCH₃,
au moyen de Cl₂ dans un solvant contenant un hydrocarbure halogéné.

La conversion du composé (2') en composé de formule (3) peut être obtenue de façon complète dans un mélange d'hydrocarbure halogéné et d'eau, mais aussi dans un hydrocarbure halogéné anhydre, sous réserve de veiller à ce que le degré d'avancement soit élevé.

Cette réaction permet de valoriser les composés de formule (2') sous-produits de réaction d'halogénation, qui n'ont pas d'application propre.

Lorsque le composé de formule (1) est le 2-acétamido trifluorométhylbenzène, le groupe Q = NHCOCH₃ reste intact au cours de la réaction d'halogénation. Il peut, si on le désire, être transformé en groupe amino par solvolyse dans un solvant approprié, tel qu'un alcool. On réalisera par exemple une méthanolyse au reflux du méthanol ou une hydrolyse.

Ainsi, l'invention a également pour objet un procédé de préparation de chloro 2-amino trifluorométhylbenzène, comprenant une étape dechloration du 2-acétamido trifluorométhylbenzène 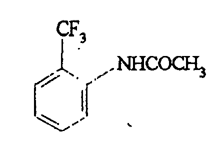 au moyen de Cl₂ suivant un procédé décrit précédemment pour former le 5-chloro 2-acétamido trifluorométhylbenzène de formule (2), 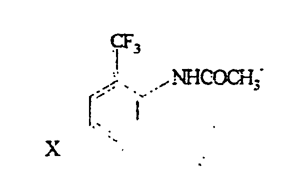 suivie d'une étape de solvolyse du composé de formule (2) obtenu.

Le procédé de mono- ou dichloration sélective selon l'invention peut être mis à profit dans une séquence réactionnelle en assurant à cette dernière un rendement global élevé en le produit désiré.

Ainsi, l'invention a pour autre objet un procédé de préparation du 3,5-dichloro trifluorométhylbenzène, comprenant une étape de dichloration du 2-amino trifluorométhylbenzène au moyen de Cl₂, les réactifs étant introduits simultanément de façon progressive dans un solvant constitué d'un mélange d'hydrocarbure halogéné et d'eau,
suivie d'une étape de diazotation-réduction du 2-amino 3,5-dichloro trifluorométhylbenzène obtenu (formule (3)), en présence d'acide hypophosphoreux HPO₂ et de nitrite de sodium NaNO₂.

Le réactif de diazotation-réduction HPO₂/NaNO₂ peut être mis en oeuvre de façon connue en soi.

De préférence, la température de réaction dans cette étape est inférieure ou égale à 30°C.

Les exemples suivants illustrent l'invention plus en détail.

### Exemple 1

On réalise la chloration du 2-aminotrifluorométhylbenzène ou ortho-trifluorométhyl-aniline (o-TFMA) dans le dichlorométhane.

Dans un réacteur, muni d'un agitateur Mixel, on charge l'o-TFMA à raison de 79,9 kg/m^{3,} et du dichlorométhane dans un rapport molaire CH₂Cl₂/o-TFMA de 31,471. Dans ce mélange à la température de 20°C, on insuffle du chlore Cl₂ en continu jusqu'à une quantité finale de 1,218 mol de Cl₂ par mol d'o-TFMA.

Après 60 minutes de réaction, la masse réactionnelle prend un aspect pâteux, dû à la précipitation de chlorhydrates des amines présentes. Cette masse est alors neutralisée par 66,1 kg/m³ de soude caustique à 30 % puis lavée à l'eau.

Une analyse de la phase organique par chromatographie en phase gazeuse conduit aux résultats suivants :

| | |
|---|---|
| TT (o-TFMA) | = 94,0 % |
| RT (3-chloro 2-aminotrifluorométhylbenzène) = RT(3-Cl) | = 0,0 % |
| RT (5-chloro 2-aminotrifluorométhylbenzène) = RT(5-Cl) | = 80,7 % |
| RT (3,5-dichloro 2-aminotrifluorométhylbenzène) = RT(3,5-diCl) | = 19,3 % |

La production du 5-chloro 2-aminotrifluorométhylbenzène est de 50 à 60 kg/m³ de mélange réactionnel.

La même analyse effectuée sur la masse réactionnelle pour des taux de transformation inférieurs montre que le dérivé 3-chloré se forme mais finit par être transformé en dérivé 3,5-dichloré. Les résultats sont rassemblés dans le tableau 1.

**Tableau 1**

| Rapport molaire Cl₂/o-TFMA | TT(o-TFMA) (%) | RT(3-Cl) (%) | RT (5-Cl) (%) | RT (3,5-diCl) (%) |
|---|---|---|---|---|
| 0,391 | 50,5 | 14,2 | 81,7 | 4,1 |
| 0,682 | 66,0 | 6,9 | 77,6 | 15,5 |
| 1,218 | 94,0 | 0,0 | 80,7 | 19,3 |

### Exemples 2 à 4 - Exemple comparatif 1

La même réaction est conduite en reproduisant le protocole de l'exemple 1 en remplaçant le dichlorométhane respectivement par du monochlorobenzène (MCB), du trifluorométhylbenzène (TFMB), de l'o-dichlorobenzène (O-DCB) et de l'eau. On vérifie dans le tableau 2 ci-après que les solvants halogénés offrent une bonne sélectivité alors que l'eau a un rôle néfaste sur la sélectivité de la chloration.

**Tableau 2**

| Exemple | 1 | 2 | 3 | 4 | Comp 1 |
|---|---|---|---|---|---|
| Solvant | CH₂Cl₂ | MCB | TFMB | o-DCB | Eau |
| Rapport molaire Cl₂/o-TFMA | 1,218 | 1,236 | 1,322 | 1,229 | 1,129 |
| TT(o-TFMA) | 94,0 | 85,0 | 90,7 | 89,4 | 97,9 |
| RT(3-Cl) | 0,0 | 0,4 | 0,3 | 0,0 | 22,1 |
| RT (5-Cl) | 80,7 | 77,2 | 74,9 | 76,3 | 61,6 |
| RT(3,5-diCl) | 19,3 | 22,7 | 25,1 | 23,7 | 16,3 |

### Exemple 5

La réaction de l'exemple 2 a été reproduite à la température de 5°C avec un rapport molaire Cl₂/o-TFMA de 1,780 avec les résultats suivants :

| | |
|---|---|
| TT (o-TFMA) | = 86,5 % |
| RT(3-Cl) | = 0,6 % |
| RT (5-Cl) | = 78,6 % |
| RT (3,5-diCl) | = 20,7 % |

### Exemples 6 à 8

On réalise la chloration en masse du 2-amino trifluorométhylbenzène (o-TFMA).

Dans un racteur muni d'un agitateur Mixel, on charge 415 g d'o-TFMA et l'on insuffle dans cette masse d'o-TFMA à 20°C du chlore Cl₂ en continu jusqu'à une quantité finale d'environ 0,3 mol de Cl₂ par mol d'o-TFMA.

A la fin de la réaction, la masse réactionnelle se compose d'un précipité et d'une phase organique. Le précipité constitué de chlorhydrates des diverses aminés présentes est filtré, lavé au monochlorobenzène, puis neutralisé à la soude caustique. Les analyses effectuées sur le précipité neutralisé et sur les phases liquides obtenues donnent les compositions suivantes,

**Tableau 3**

| | o-TFMA | 3-Cl | 5-Cl | 3,5-diCl |
|---|---|---|---|---|
| Précipité (g) | 125,4 | 2,7 | 15,2 | 0,3 |
| Filtrat (g) | 126,3 | 27,9 | 73,5 | 3,3 |
| Lavage MCB (g) | 38,5 | 8,6 | 21,9 | 0,9 |
| Total (g) | 290,2 | 39,2 | 110,6 | 4,5 |
| | (1,80 mol) | (0,200 mol) | (0,565 mol) | (0,020 mol) |

soit le bilan molaire suivant :

| | |
|---|---|
| TT (o-TFMA) | = 30,3 % |
| RT(3-Cl) | = 25,5 % |
| RT (5-Cl) | = 72,0 % |
| RT (3,5-diCl) | = 2,5 % |

Les dérivés monohalogénés se trouvent principalement dans la partie soluble, vraisembablement sous forme de base libre.

Deux autres essais donnent les résultats rassemblés dans le tableau 4 suivant :

**Tableau 4**

| Exemple | 7 | 8 |
|---|---|---|
| Rapport molaire Cl₂/o-TFMA | 0,340 | 0,297 |
| TT(o-TFMA) | 32,0 | 26,8 |
| RT(3-Cl) | 27,5 | 26,7 |
| RT (5-Cl) | 69,3 | 71,6 |
| RT (3,5-diCl) | 3,2 | 1,6 |

Dans ces trois exemples, la productivité en 2-amino 5-chloro trifluorométhylbenzène est de l'ordre de 300 kg/m³ de mélange réactionnel.

### Exemple 9 :

On réalise la chloration du 2-acétamido trifluorométhylbenzène selon l'invention.

Pour les besoins de cet exemple, le produit de départ a été synthétisé par acétylation de l'orthotrifluorométhyl aniline par l'anhydride acétique à 60°C, dans le monochlorobenzène, de façon connue en soi, suivant l'équation :

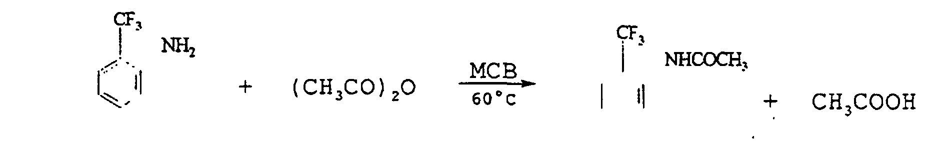

La chloration du 2-acétamido trifluorométhylbenzène a ensuite été effectuée en reproduisant le protocole de l'exemple 2 en chauffant à la température de 80°C.

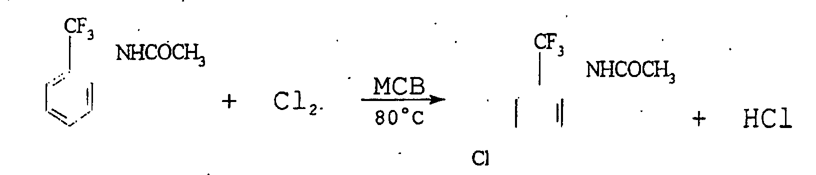

Le 5-chloro 2-acétamido trifluorométhyl benzène est formé avec un RT de 66,4 %.

On peut isoler le 5-chloro 2-amino trifluorométhylbenzène correspondant après méthanolyse à reflux (5 heures au reflux du méthanol) puis distillation de l'acétate de méthyle formé et du méthanol en excès.

### Exemple 10

### Synthèse du 3,5-dichloré trifluorométhyl benzène

### A - Préparation du 2-amino 3,5-dichloro trifluorométhylbenzène

Dans un mélange de 250 g d'eau et 250 g de monochlorobenzène, à température ambiante (20 à 25°C), on introduit simultanément 100 g d'orthotrifluorométhylaniline à raison de 0,54 g/min et 305 g de chlore à raison de 0,30 g/min (rapport molaire Cl₂/o-TFMA = 2.111).

En fin de réaction, la masse réactionnelle constituée de 380 g de phase organique et 300 g de phase aqueuse est neutralisée avec 208 g de lessive de soude à 27 % en poids de NaOH (rapport molaire NaOH/Cl₂ = 1,082), puis lavée à l'eau.

La phase organique est concentrée et distillée sous vide (7 à 11 mbar) pour isoler le 2-amino 3,5-dichlorotrifluométhylbenzène.

Les résultats sont les suivants :

| | |
|---|---|
| TT (o-TFMA) | = 100,0 % |
| RT(3-Cl) | = 0,2 % |
| RT (5-Cl) | = 0,4 % |
| RT (3,5-diCl) | = 76,6 % |

### B - Diazotation-réduction au 2-amino dichlorotrifluorométhylbenzène

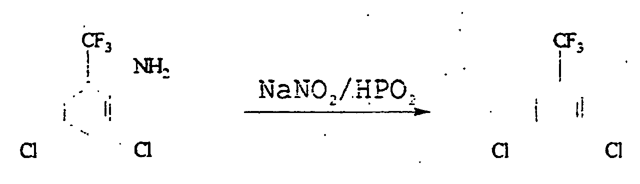

Dans un réacteur de 500 ml, on charge 49,3 g d'eau, 15,8 g d'acide hypophosphoreux à 50 % (0,120 mol) et 0,13 g de sulfate de cuivre pentahydraté (0,52.10⁻³ mol).

On ajoute ensuite à température ambiante 19,5 g d'acide sulfurique concentré (0,195 mol).

23 g de 2-amino 3,5-dichloro trifluorométhyl benzène (0,100 mol) sont alors ajoutés en 25 minutes à 25°C environ, puis on ajoute 17,1 g d'une solution à 41,5 % de nitrite de sodium. La température est maintenue à 30°C maximum au cours de cette addition exothermique, puis pendant 1 heure à 25°C. La phase organique est ensuite décantée et lavée à l'eau.

On obtient finalement un mélange constitué à 22.8 % d'aniline et à 72,3 % de 3,5-dichloro trifluorométhylbenzène.

Le taux de transformation du produit de départ a été de 78,2 % et le rendement réel en dichlorotrifluorométhylbenzène est de 74,0 %.

On remarque que lorsque la température de réaction est de 35°C au lieu de 25°C, on obtient un mélange de 65,2 % d'aniline et 29,6 % de dichloro trifluorométhyl benzène (rendement réel = 28,9 % ; taux de transformation 40,5 %).

## Revendications

1. Procédé de préparation de dérivés du 2-amino ou du 2-acétamido trifluorométhylbenzène chlorés en position 5 du cycle benzénique, comprenant une étape de chloration d'un composé de formule (1) 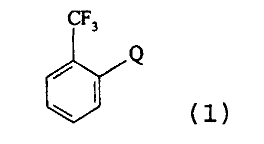 dans laquelle Q représente un groupe NH₂ éventuellement sous forme d'un sel d'addition avec un acide tel que l'acide chlorhydrique, ou un groupe NHCOCH₃,
au moyen de Cl₂,
dans un solvant choisi parmi un hydrocarbure halogéné anhydre, un mélange d'eau et d'hydrocarbure halogéné, et le composé de formule (1) lui-même,
pour donner majoritairement un composé de formule (2) ou (3) 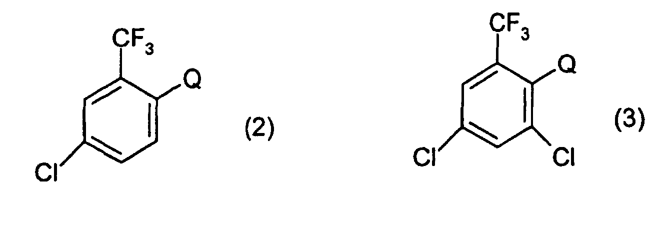 formules dans lesquelles Q est tel que défini ci-dessus.

2. Procédé selon la revendication 1 dans lequel ledit solvant est un solvant halogéné anhydre et le produit majoritairement obtenu est le composé de formule (2).

3. Procédé selon la revendication 2 dans lequel le rapport molaire de la quantité de Cl₂ introduite à la quantité de composé de formule (1) est de 1 à 2.

4. Procédé selon la revendication 2 ou 3, dans lequel la réaction d'halogénation est conduite jusqu'à ce que le taux de transformation du composé de formule (1) soit d'au moins 80 %.

5. Procédé selon la revendication 1, dans lequel ledit solvant est constitué du composé de formule (1) lui-même et le produit majoritairement obtenu est le composé de formule (2).

6. Procédé selon la revendication 5, dans lequel le rapport molaire de la quantité de Cl₂ introduite au composé de formule (1) est de 0,1 à 0,5.

7. Procédé selon la revendication 1 dans lequel on obtient majoritairement le composé de formule (3) en introduisant le composé de formule (1) de façon progressive, de même que le Cl₂, dans un solvant constitué d'un mélange d'hydrocarbure halogéné et d'eau.

8. Procédé selon la revendication 7, dans lequel ledit solvant contient au moins 20 % en poids d'hydrocarbure halogéné.

9. Procédé selon la revendication 7 ou 8, dans lequel le rapport molaire de la quantité de Cl₂ introduite à la quantité de composé de formule (1) introduite est de 1,5 à 2,5.

10. Procédé selon l'une quelconque des revendications 1 à 9, dans lequel la température du milieu réactionnel est de 5 à 80°C.

11. Procédé selon la revendication 10, dans lequel Q, dans la formule (1), représente un groupe NH₂ éventuellement sous forme d'un sel d'addition, et la température du milieu réactionnel est de 5 à 50°C.

12. Procédé selon la revendication 10, dans lequel Q, dans la formule (1), représente un groupe NHCOCH₃ et la température du milieu réactionnel est de 70 à 80°C.

13. Procédé de préparation d'un dérivé dichloré du 2-amino ou 2-acétamido trifluorométhylbenzène, comprenant une étape de chloration d'un composé de formule (2') 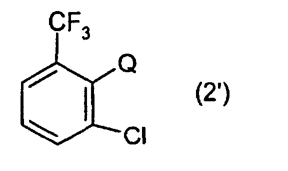 dans laquelle Q représente un groupe NH₂ éventuellement sous forme d'un sel d'addition avec un acide tel que l'acide chlorhydrique, ou un groupe NHCOCH₃,
au moyen de Cl₂ dans un solvant contenant un hydrocarbure halogéné.

14. Procédé selon la revendication 13, dans lequel le solvant contient en outre de l'eau.

15. Procédé de préparation d'halogéno 2-amino trifluorométhylbenzène, comprenant une étape de chloration du 2-acétamido trifluorométhylbenzène 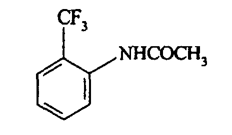 au moyen de Cl₂, suivant l'une quelconque des revendications 1 à 12, pour former le 5-chloro 2-acétamido trifluorométhylbenzène de formule (2) 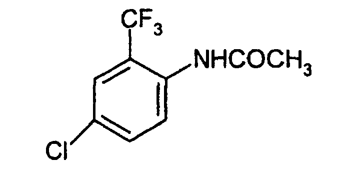 suivie d'une étape de solvolyse du composé de formule (2) obtenu.

16. Procédé de préparation du 3,5-dichloro trifluorométhylbenzène, comprenant une étape de dichloration du 2-amino trifluorométhylbenzène au moyen de Cl₂, suivant un procédé selon l'une quelconque des revendications 7 à 9,
suivie d'une étape de diazotation-réduction du 2-amino 3,5-dichloro trifluorométhyl benzène de formule (3) obtenu, en présence d'acide hypophosphoreux^{H3P02} et de nitrite de sodium NaNO₂.

17. Procédé selon la revendication 16, **caractérisé en ce que** la température dans l'étape de diazotation-réduction est inférieure ou égale à 30°C.

## Patentansprüche

1. Verfahren zur Herstellung von in 5-Position des Benzolrings chlorierten Derivaten von 2-Amino- oder 2-Acetamido-trifluoromethylbenzol, umfassend einen Schritt der Chlorierung der Verbindung der Formel (1) 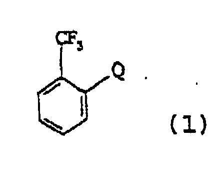 wobei Q eine NH₂-Gruppe darstellt, gegebenenfalls in Form eines Additionssalzes mit einer Säure wie Salzsäure, oder eine Gruppe NHCOCH₃ darstellt,
mit Hilfe von Cl₂,
in einem Lösungsmittel, das ausgewählt wird aus einem wasserfreien halogenierten Kohlenwasserstoff, einer Mischung von Wasser und einem halogenierten Kohlenwasserstoff, und der Verbindung (1) selbst,
um hauptsächlich eine Verbindung der Formel (2) oder (3) herzustellen 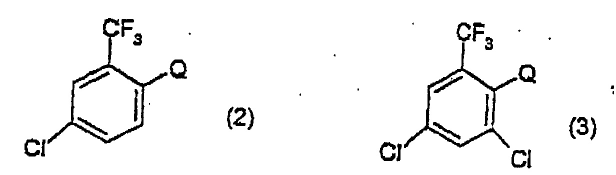 wobei in diesen Formeln Q wie oben definiert ist.

2. Verfahren nach Anspruch 1, wobei das Lösungsmittel ein wasserfreies halogeniertes Lösungsmittel ist und das hauptsächlich erhaltene Produkt die Verbindung der Formel (2) ist.

3. Verfahren nach Anspruch 2, wobei das molare Verhältnis der Menge von eingeführtem Cl₂ zur Menge der Verbindung der Formel (1) 1 bis 2 beträgt.

4. Verfahren nach Anspruch 2 oder 3, wobei die Halogenierungsreaktion bis zu einem Umsatz der Verbindung der Formel (1) von mindestens 80 % durchgeführf wird.

5. Verfahren nach Anspruch 1, wobei das Lösungsmittel die Verbindung der Formel (1) selbst ist und das hauptsächlich erhaltene Produkt die Verbindung der Formel (2) ist.

6. Verfahren nach Anspruch 5, wobei das molare Verhältnis der Menge von eingeführtem Cl₂ zur Verbindung der Formel (1) 0,1 bis 0,5 beträgt.

7. Verfahren nach Anspruch 1, wobei man hauptsächlich die Verbindung der Formel (3) erhält, indem die Verbindung der Formel (1) genau so wie Cl₂ allmählich in ein Lösungsmittel gegeben wird, das aus einer Mischung von halogeniertern Kohlenwasserstoff und Wasser besteht.

8. Verfahren nach Anspruch 7, wobei das lösungsmittel mindestens 20 Gew.-% halogenierten Kohlenwasserstoff enthält.

9. Verfahren nach Anspruch 7 oder 8, wobei das molare Verhältnis der Menge von eingeführtem Cl₂ zur Menge der eingeführten Verbindung der Formel (1) 1,5 bis 2,5 beträgt.

10. Verfahren nach einem der Ansprüche 1 bis 9, wobei die Temperatur des Reaktionsmediums 5 bis 80 °C beträgt.

11. Verfahren nach Anspruch 10, wobei Q In der Formel (1) eine NH₂-Gruppe darstellt, gegebenenfalls in Form eines Säureadditionssalzes, und die Temperatur des Reaktionsmedlums 5 bis 50 °C beträgt.

12. Verfahren nach Anspruch 10, wobei Q in der Formel (1) eine NHCOCH₃-Gruppe darstellt und die Temperatur des Reaktionsmediums 70 bis 80 °C beträgt.

13. Verfahren zur Herstellung eines dichlorierten Derivats von 2-Aminooder 2-Acetamido-trifluoromethylbenzol, umfassend einen Schritt der Chlorierung einer Verbindung der Formel (2') 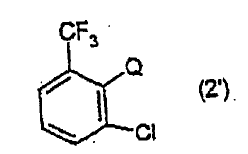 wobei Q eine NH₂-Gruppe, gegebenenfalls in Form eines Additionssalzes mit einer Säure wie Salzsäure, oder eine NHCOCH₃-Gruppe darstellt,
mit Hilfe von Cl₂ in einem Lösungsmittel, das einen halogenierten Kohlenwasserstoff enthält.

14. Verfahren nach Anspruch 13, wobei das Lösungsmittel außerdem Wasser enthält.

15. Verfahren zur Herstellung von Halogeno-2-aminotrifluoromethylbenzol, umfassend einen Schritt der Chlorierung von 2-Acetamido-trifluoromethylbenzol 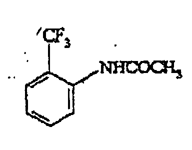 mit Hilfe von Cl₂ noch einem der Ansprüche 1 bis 12 zur Herstellung von 5-Chloro-2-acétamido -trifluoromethylbenzol der Formel (2) 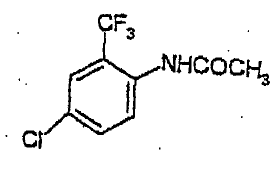 gefolgt von einem Solvolyseschriff der erhaltenen Verbindung der Formel (2).

16. Verfahren zur Herstellung von 3,5-Dichloro-trifluoromethylbenzol, umfassend einen Schritt der Dichlorierung von 2-Aminotrifluoromethylbenzol mit Hilfe von Cl₂ nach einem Verfahren nach einem der Ansprüche 7 bis 9
gefolgt von einem Schritt der Diazotierung-Reduktion des erhaltenen 2-Amino-3,5-dichloro-trifluoromethylbenzol der Formel (3) In Gegenwart von hypophosphoriger Säure **H3P02** und Natriumnitrit NoNO₂.

17. Verfahren nach Anspruch 16, dadurch charakterisiert, dass die Temperatur im Diazotierungs-Reduktions-Schritt kleiner oder gleich 30 °C ist.

## Claims

1. Process for preparing 2-amino- or 2-acetamidotrifluoromethylbenzene derivatives chlorinated in position 5 of the benzene ring, comprising a step of chlorinating a compound of formula (1) 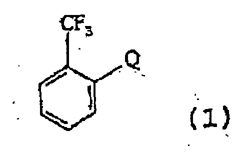 in which Q represents an NH₂ group optionally in the form of an addition salt with an acid such as hydrochloric acid, or an NHCOCH₃ group,
using Cl₂,
in a solvent chosen from an anhydrous halogenated hydrocarbon, a mixture of water and of halogenated hydrocarbon and the compound of formula (1) itself,
to give mainly a compound of formula (2) or (3) 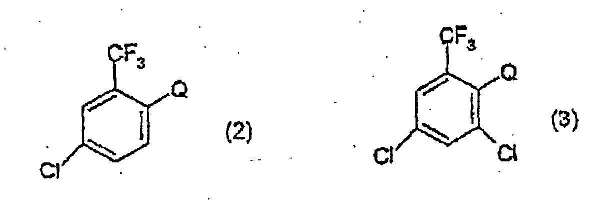 in which formulae Q is as defined above.

2. Process according to Claim 1, in which the said solvent is an anhydrous halogenated solvent and the product mainly obtained is the compound of formula (2).

3. Process according to Claim 2, in which the molar ratio of the amount of Cl₂ introduced to the amount of compound of formula (1) is from 1 to 2.

4. Process according to Claim 2 or 3, in which the halogenation reaction is carried out until the degree of conversion of the compound of formula (1) is at least 80%.

5. Process according to Claim 1, in which the said solvent consists of the compound of formula (1) itself and the product mainly obtained is the compound of formula (2).

6. Process according to Claim 5, in which the molar ratio of the amount of Cl₂ introduced to the compound of formula (1) is from 0.1 to 0.5.

7. Process according to Claim 1, in which the compound of formula (3) is mainly obtained by introducing the compound of formula (1) gradually, along with the Cl₂, into a solvent consisting of a mixture of halogenated hydrocarbon and water.

8. Process according to Claim 7, in which the said solvent contains at least 20% by weight of halogenated hydrocarbon.

9. Process according to Claim 7 or 8, in which the molar ratio of the amount of Cl₂ introduced to the amount of compound of formula (1) introduced is from 1.5 to 2.5.

10. Process according to any one of Claims 1 to 9, in which the temperature of the reaction medium is from 5 to 80°C.

11. Process according to Claim 10, in which Q, in formula (1), represents an NH₂ group optionally in the form of an addition salt, and the temperature of the reaction medium is from 5 to 50°C.

12. Process according to Claim 10, in which Q, in formula (1), represents an NHCOCH₃ group and the temperature of the reaction medium is from 70 to 80°C.

13. Process for preparing a dichloro derivative of 2-amino- or 2-acetamidotrifluoromethylbenzene, comprising a step of chlorinating a compound of formula (2') 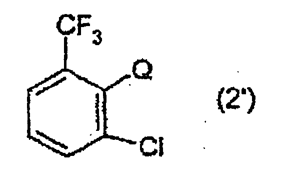 in which Q represents an NH₂ group optionally in the form of an addition salt with an acid such as hydrochloric acid, or an NHCOCH₃ group, using Cl₂ in a solvent containing a halogenated hydrocarbon.

14. Process according to Claim 13, in which the solvent also contains water.

15. Process for preparing halo-2-aminotrifluoromethylbenzene, comprising a step of chlorinating 2-acetamidotrifluoromethylbenzene 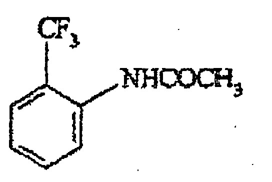 using Cl₂, according to any one of Claims 1 to 12, to form the 5-chloro-2-acetamidotrifluoromethylbenzene of formula (2) 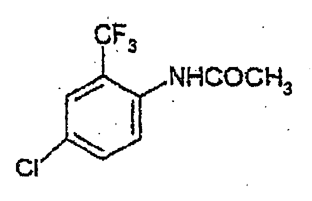 followed by a step of solvolysis of the compound of formula (2) obtained.

16. Process for preparing 3,5-dichlorotrifluoromethylbenzene, comprising a step of dichlorinating 2-aminotrifluoromethylbenzene using Cl₂, according to a process according to any one of Claims 7 to 9,
followed by a step of diazotization-reduction of the 2-amino-3,5-dichlorotrifluoromethylbenzene of formula (3) obtained, in the presence of hypophosphorous acid **H3P02** and sodium nitrite NaNO₂.

17. Process according to Claim 16, **characterized in that** the temperature in the diazotization-reduction step is less than or equal to 30°C.
